# EUROPEAN PATENT SPECIFICATION

(11) **EP 0 223 763 B1**
(45) Date of publication and mention of the grant of the patent: **05.05.1993**
(21) Application number: 86850399.6
(22) Date of filing: 21.11.1986
(51) Int. Cl.: A61F 2/02, A61M 31/00

(54) **An artificial gland for implantation in the human body**
Künstliche Drüse zur Implantation in den menschlichen Körper
Glande artificielle pour implantation dans le corps humain

(30) Priority: 22.11.1985 NO 854668
(43) Date of publication of application: 27.05.1987
(73) Proprietor: INDUSTRIKONTAKT ING. O. ELLINGSEN & CO:, N-6900 Floro (NO)
(72) Inventor: Ellingsen, Olav, N-6900 Floro (NO)
(74) Representative: Graudums, Valdis

(56) References cited:
- EP-A- 0 089 548
- FR-A- 2 195 461
- US-A- 4 364 385

## Description

The present invention relates to an artificial gland, e.g. an artificial pancreas, for implantation in the body of a patient suffering from a disease that may be treated/kept in check by continuous medication, and where said medication is adiministered to the patient's body as a function of changes of the blood composition. As an example of such a disease, we may pention diabetes. Patients suffering from diabetes need injections of insulin at fixed timed to control the disease that is considered incurable. Such a device or artificial gland comprises two main members, a reservoir for medication, which reservoir is filled by a syringe through the skin, and a membrane provided on said reservoir, and a membrane enclosing a sponge having an opped celled structure and, in turn, being connected with said reservoir by a hose. When a foreign body is implanted in the human body a tissue fluid will be formed around said foreign body. The composition of said fluid will change dependent on the disease to be treated and said change will cause an osmotic pressure difference between said membrane and the medication enclosed in said sponge, or it will influence said membrane by osmotic pressure on substance embedded therein. The molecules providing said osmotic pressure will migrate through said membrane and increase the pressure in said sponge or in a void between the sponge and the membrane causing delivery of medicament to the tissue fluid. The technique of delivery will vary with individual presentation of illness and by reversible mechanical changes in said membrane, e.g. by membrane expansion pores in the membrane will be opened causing medicament to flow into the tissue fluid and to reverse said membrane expansion.

In order to explain the present invention it will now be disclosed with reference to its utilization for persons suffering from diabetes mellitus. To day, this disease is essentially controlled by administration of insulin to the body by injections. This means that a patient suffering from this disease can keep it in check. The total amount of insulin being necessary for one day, however, being injected in one or two doses, considerable disadvantages arise. The patient has to keep a strict diet which has to be taken with regular intervals during the day, usually with intervals of 3 hours, to keep the blood sugar within an acceptable level. Since the body's consumption of blood sugar is influenced by several factors it may happen that the blood sugar gets too low, resulting in a so called "feeling". If this state is to heavy or too long-lasting it may cause brain damage. If the blood sugar gets too high the same symptoms as with incurable diabetes reoccur and these may be fatal if left to develop. Furthermore, the so called "late effects" of diabetes represent a serious problem. Among others, they comprise danger of blindness, problems with veins, etc. A patient suffering from diabetes is also far more vulnerable as regards infectious diseases than healthy people.

In order to remedy these circumstances extensive research is done to day, both as regards this and other diseases making the patient dependent on medicamentation. Tn this connection, so called "insulin pumps" were developed. A patient may adhere such a pump to his body for receiving supply of insulin by the aid of a cannula uniformely all the day long. Before a meal the amount of insulin may be increased by activating the pump. Also, an implantable pump was designed which functions along the same principles but is filled by the aid of a syringe approximately once a week through the skin. Said pump is provided with a membrane. Activation of said pump before a meal may be achieved by the aid of an ultrasonic transmitter. All these pumps resulted in considerable improvement of the insulin supply to patients and of the general state of health of those using the pumps.

The pumps mentioned, however, are nothing but large syringes containing insulin for several day's consumption. They are not controlled in dependence on the momentary blood sugar level which is the factor determining the demand for insulin. Furthermore, they have great practical disadvantages, Pumps secured to the outside of an active person's body are rather exposed to blows, to being torn off, etc. and careful maintenance is needed. Also, the cannula into the human body must be replaced sometimes. The above mentioned implantable pump does not show these disadvantages, but it has no function of controlling the blood sugar. Otherwise, it functions in the same manner as the first mentioned with a battery drive forming the source of energy, which involves a surgical intervention whenever the battery has to be replaced.

Additionally, implantable medicament pumps/devices are known from US-PS Nos. 4 073 292, and 3 923 060 and FR-A-2195461. The first mentioned specification relates to an implantable pancreas controlling the insulin supply as a function of the content of sugar in urine. The detection of changes of the sugar level in urine is photoelectric by detection of the passage of light through filtered urine with Benedict's solution added. This patent has the disadvantage that two fluids have to be fed to the body: the medicament, i.e. insulin in the present case, and an activator liquid for the urine. This is a disadvantage in the case of the disease in question, two kinds of injections being necessary with the hazard of confusing the reservoirs which may have fatal consequences. Also, the requirement of filtering urine will necessitate exchange of the filter, which in this case will imply a surgical intervention. Additionally, it is a fact that the detection of any possible sugar content in urine is not a satisfactory manner of detecting the sugar content of blood because, when sugar is precipitated in urine, the sugar level is already far too high in the patient's blood. Such a control of the disease may keep the blood sugar under control, only this will be at a far too high level. Besides, the operation of said device is exclusively by battery.

The last mentioned patent discloses an implantable pump wich is, primarily, intended for use in connection with heart diseases by detecting changes in blood pressure, electric signals, and chemical changes in the body. Apart from the sensor detecting changes of blood pressure, there is no disclosure of how the remaining parameters are to be detected. It is only stated that they can be detected. The operation of the device, like that of the above mentioned devices, is by battery of a kind known from "pace-makers".

It is an object of this invention to remedy some of these disadvantages of pumps of the mentioned kind, which means that the following requirements should be fulfilled:
1. The artificial "pancreas" (in the following called gland) is provided with a reservoir that is filled with insulin by the aid of a syringe piercing the skin and a membrane that is provided on said reservoir and covers the insulin container of the gland. Known per se.
2. Said reservoir is provided with a bleeding unit for injecting insulin into the human body, said unit "sensing" the demand for insulin of the body at any time.
3. Said gland must be 100% reliable and independent of any special sources of energy for operation.
4. A safety means closing in case of any conceivable fault.

This invention relates to an artificial gland for implantation in the human body comprising a medicament reservoir intended to be filled with a medicament solution by the aid of a syringe, wherein said reservoir is connected via a passage to a casing, the wall of which is completely or partly comprised of a semi-permeable membrane causing changes of pressure by osmosis for delivery of a medicament, characterized in that said passage includes a pressure activated valve preventing uncontrolled amount of medicament to flow into the body, that a sponge for absorbing medicament solution is enclosed in said casing, said body occupying only part of the casing void and being defined against the remaining portion of said void by a tight film, from which channels with outwardly opening one-way valves lead out through the semi-permeable membrane casing wall, the space between said casing wall and said tight film, in use, being filled with body fluid.

Alternatively the casing wall includes a rigid semi-permeable membrane and a flexible hose leads from the capillary tube end inside said casing to the opposite casing wall, said flexible hose enclosing a plug having a blind centre bore, one end of which extends out through said casing wall, while its other end opens into a transverse bore covered by said flexible hose.

In one embodiment of the invention said casing wall consists of a rigid semipermeable membrane permitting an osmotic pressure to build up in the tissue fluid in the space between said casing wall and tight film to force liquid medicament out of said sponge.

In another embodiment part of said casing wall consists of a resilient semi-permeable membrane provided with a portion pressing against the film enclosing said sponge and liberating medicament solution therefrom, when said membrane collapses due to reduction of the osmotic pressure in the body fluid in the space between said casing wall and said tight film.

In a further embodiment a rigid casing is provided with a casing wall that is perforated with small openings which at one end are closed by a rigid, semi-permeable membrane, a resilient balloon being attached at the zone of transition between said perforated casing wall and said membrane, which balloon is biased into sealing contact with the inside of said perforated casing wall by the aid of the spring actions of a sponge provided inside said balloon.

The invention is now to be disclosed in detail with reference to the drawings:

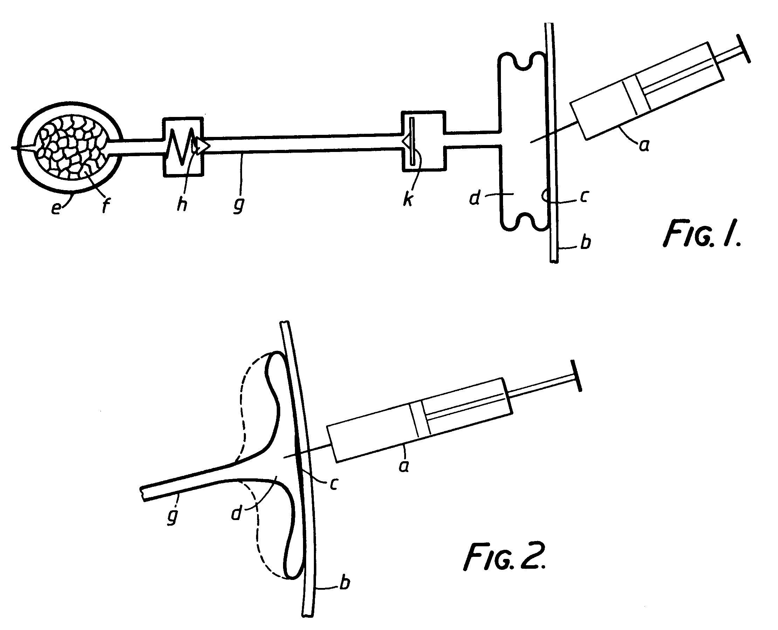

Figure 1 shows the main elements of the gland, a being a syringe which fills a reservoir d through the skin b and a membrane c. Said reservoir is, via a hose g, connected with a semipermeable membrane e enclosing a sponge f which has an open celled structure. In three of the embodiments a check valve h is provided in hose g. Furthermore, a mechanical safety means k, as mentioned under point 4 above, is provided. It functions to close the liquid flow through hose g in case of any physical pressure on reservoir d. Hose g is dimensioned with an opening that is so small that even if safety means k should fail, the friction of the liquid flow in case of pressure on reservoir d will brake the liquid flow to prevent excessive liquid to sponge f.

Figure 2 is a sectional view of reservoir d which is arranged to expand when insulin is supplied to it and collapses when it is emptied. Said collapse is used to emit a mechanical signal indicating an empty reservoir.

Membrane e may be designed in four different manners:
In Figure 3 it is a polymer having pore openings corresponding to the glucose (sugar) molecules which are considerably smaller than the insulin molecules.

In Figure 4 said membrane is a polymer having pore openings with outwards facing lips only permitting liquid flow in one direction.

In Figure 5 said membrane is a polymer with an encased enzyme/substance which reacts by expansion when contacted with glucose and corresponding contraction when the glucose disappears.

Figure 6 shows an embodiment wherein membrane e is a non-compressible semipermeable membrane enclosing sponge f, but with an interspace between said sponge and membrane. Sponge f is tightly enclosed by a resilient tight plastic film l having tentacles m which only permit liquid flow in one direction, i. e. outwards from said sponge. Said tentacles extend through said semipermeable membrane. Check valve h is placed at the outlet of hose g and safety means k is placed at the inlet of said hose which is designed as a conical sleeve of stainless steel, ceramics, or some other rigid synthetic material.

Figure 7 shows another embodiment of the membrane arrangement of the embodiment in Figure 6. The non-compressible semipermeable membrane forms a heisphere about sponge f. Film l forms a "lid" between said hemisphere and sponge. Around sponge f a casing n of a rigid synthetic material is provided, and on this casing tentacles o are provided to permit liquid flow in but one direction, i.e. out of the sponge. This embodiment is equal to that of Figure 6 as regards remaining features.

The effect and mode of operation with the mentioned four kinds of membranes will be disclosed below.

With reference to Figure 1 the function of the gland is as follows:
After implantation a tissue fluid will form around said semipermeable membrane as a protection for the adjacent tissue. This tissue fluid will penetrate through said semipermeable membrane to fill the interspace between said sponge f with film l and said membrane until an osmotic equilibrium is achieved between fluid outside and inside said membrane. The composition of said tissue fluid will vary as a function of the pathological picture present. In the case of diabetes this means that the content of glucose in said fluid will increase at the same pace as the blood sugar.

When there is an increase of the glucose content of the tissue fluid around said semipermeable membrane this will cause an osmotic pressure difference between the tissue fluid outside said membrane and the fluid in the interspace between said sponge and membrane. The pores of said membrane are dimensioned in such a manner that the sugar molecules will migrate through said membrane and into said sponge. This will cause an increase of pressure in said interspace and said membrane not being resilient it will cause said sponge to be compressed. This will, in turn, cause the insulin that entered said sponge through hose g to be forced out into the patient's body along tentacles m, since check valve h prevents the insulin from being forced back into reservoir d. When the content of glucose outside said membrane decreases as a result of the insulin reducing the glucose content in tissue fluid, the opposite will take place, i.e. the sugar molecules migrate out of said interspace forming an underpressure in this case, which will cause said sponge to expand and new insulin to be sucked into it.

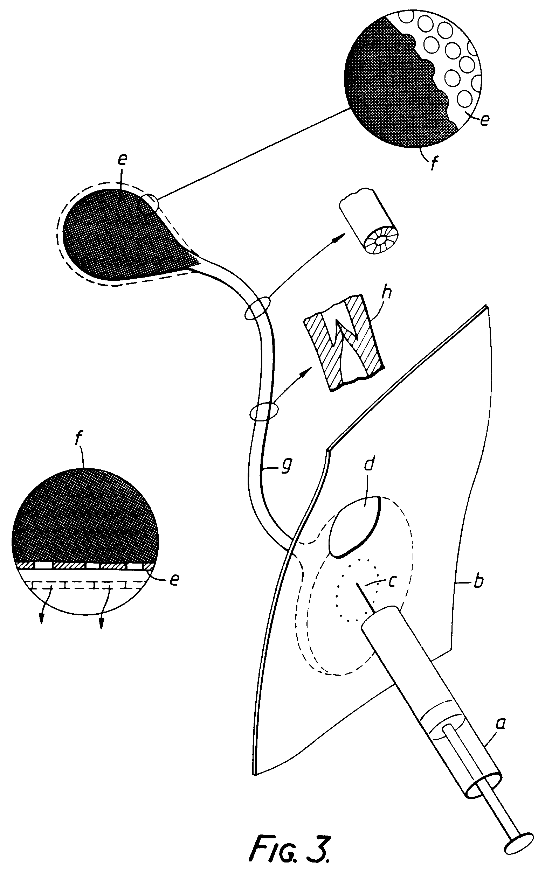

In Figure 3 said sponge is enclosed by a resilient semipermeable membrane which is dimensioned so as to let only sugar molecules pass through in a resting position, but not the insulin in said sponge. This is possible because insulin molecules are considerably larger than sugar molecules.

When the concentration of glucose in the tissue fluid around the membrane increases the sugar molecules will migrate into said sponge. Due to check valve h an excessive pressure will build up in the sponge, and the membrane will expand. This will cause the pores to expand causing both insulin and the migrated sugar molecules to migrate outwards into the tissue fluid. When the glucose content is decomposed due to the influence from insulin said sponge will contract and more insulin will be sucked into it.

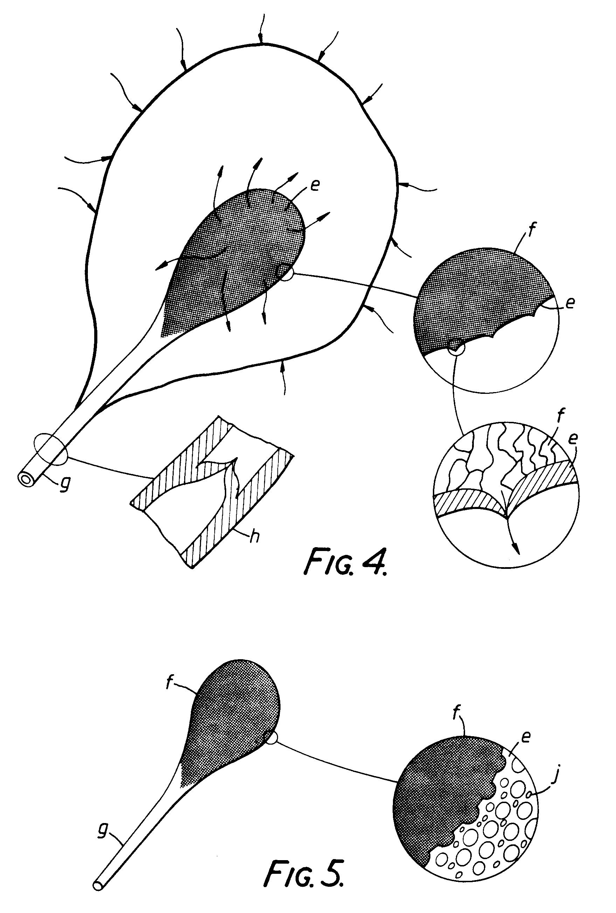

In Figure 4 the membrane is a tight film provided with openings and lips only permitting liquid flow in one direction, i.e. towards the tissue fluid. In this embodiment a check valve is also provided in the hose. Said membrane is, in this case, positioned in a closed area in the body where the liquid collected around said membrane is provided with a film.

When the content of glucose in the body increases glucose will migrate into said closed collected fluid. Here, an excessive pressure will build up in relation to the pressure in the sponge, and the membrane will be compressed. Due to compression insulin (or other medicaments) will be forced out through the "one way pores". When the pressure decreases said membrane will expand and suck insulin from said reservoir.

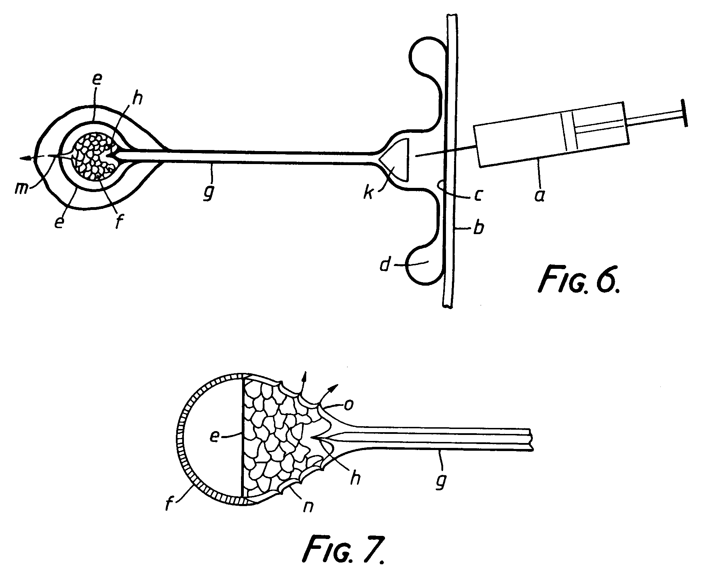

In Figure 5 an enzyme/reactive substance j is "baked" into the membrane. In case of contact with glucose a change of phases, an expansion of the substance will take place, and the membrane as such will expand. "Said "change of phases" may, also, be caused by osmotic forces sugar molecules migrating into said enzyme. Said expansion will cause the non-permeable pores to open up sufficiently to let insulin migrate to the tissue fluid. This expansion of pores is an analoge to the pores in a balloon that is inflated. In a non inflated condition said pores may be quite tight, whereas they can let out air or some other medium, e.g. water, in a certain state of inflation. When the glucose is reduced the phase condition of said substance will be reversed, the pores are closed and new insulin will enter the sponge. In this embodiment no check valve is needed.

The osmotic effect discussed here may, inter alia, be observed in patients suffering from uncontrolled diabetes. When the glucose content of the body increases the sugar molecules will also migrate into the eye ball, an osmotic equilibrium forming in the whole organism. In treatment of a very advanced diabetes where the glucose content is very high, e.g. more than 30 mmol/litre, it will be observed after an intravenous injection of insulin that there is a delay in the reduction of glucose in the eye ball, the eye ball literally bulging out due to the osmotic pressure there in relation to the remaining tissue fluids. After a while such bulging will recede.

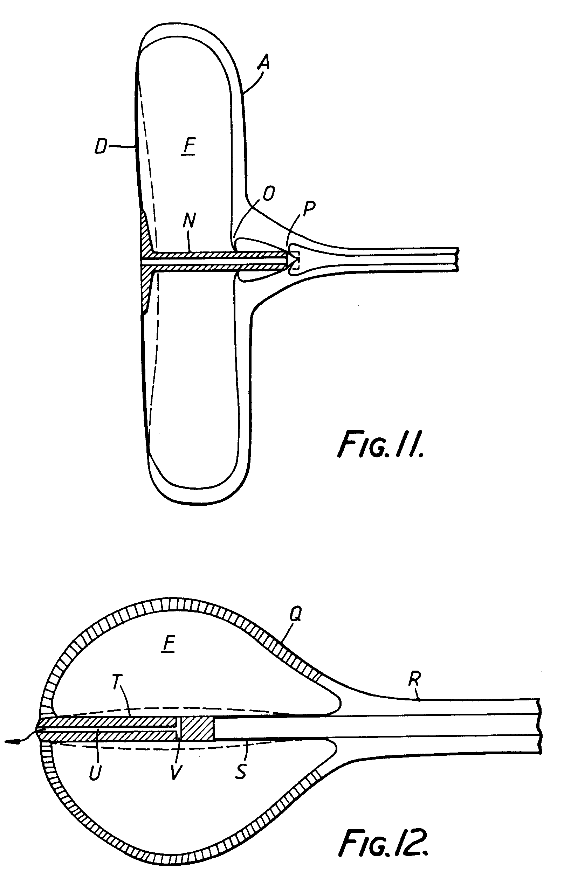

Figure 10 shows an embodiment with functions as disclosed in connection with Figure 1. In Figure 7 another embodiment of the sponge and the semipermeable membrane is shown. Here, the influence of pressure is on film l between membrane e and sponge f. Insulin is, thus, forced out through one way ports o.

We have disclosed the principles of the gland which functions as a result of glucose passing through the membrane, i.e. inverted osmosis. The principle may, also, be adapted for conventional osmosis, i.e. the membrane is dimensioned so as to prevent glucose from passing through, whereas all smaller molecules may pass. When an increase of glucose occurs in the human body migration of low molecular particles will occur through a membrane towards the tissue fluid, and these particles will seek to "dilute" the tissue fluid with its content of glucose. This principle means that an underpressure is to be achieved in the gland contrasting with the above principle where an excessive pressure is built in the gland.

This is disclosed in more detail with reference to Figures 8, 9, 10, 11, 12, 13, and 14 showing various embodiments of the "sensor portion", i.e. the portion of the gland that is placed in tissue fluid and where the membrane is provided. The reservoir for the insulin supply and the filling operation is the same as for the above disclosed devices. In order to differentiate from Figures 1-7 capital letters are used for reference symbols.

Fig. 8 shows the "sensor portion" A being a casing of non-compressible plastic. There is one or several ports B for insulin delivery. Ports B are normally closed by flaps C. At the end of casing A there is a membrane D shutting off glucose. Membrane D has a thickening E in the middle. When tissue fluid passes out from the collection of fluid in F due to increase of glucose outside said "sensor portion" an underpressure is caused in F. Thus, membrane D collapses, and E urges against a balloon G that is filled with a sponge having an open celled structure H. The insulin in said sponge will be forced out through ports B, since check valve I prevents a return flow to the reservoir. When the glucose content decreases in the body the opposite effect will occur, liquid passes into F, membrane D is expanded, the balloon expands and insulin is sucked from said reservoir.

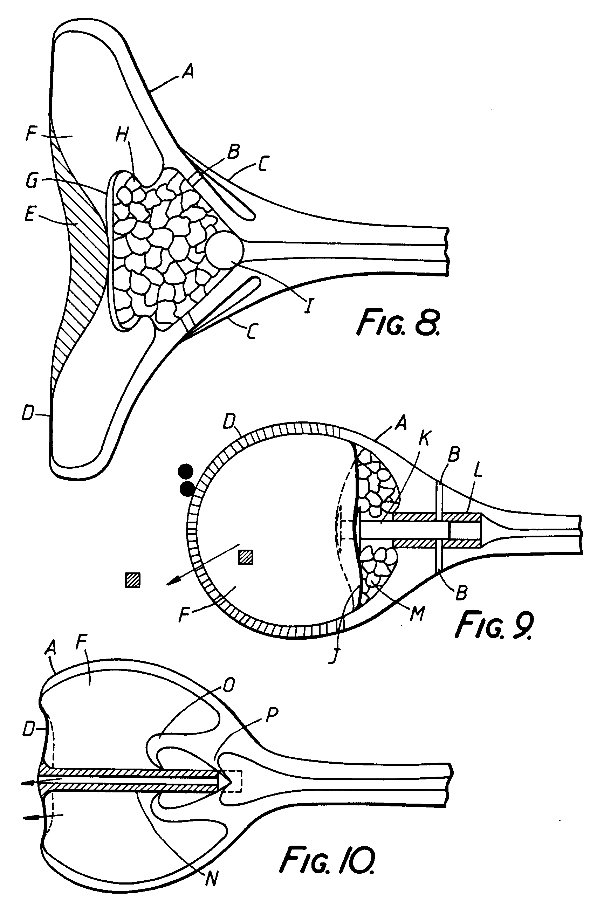

Figure 9 shows a "sensor portion" where casing A comprises a non-compressive membrane D. In space F where fluid collects a resilient film J is provided. A piston K extending into a cylinder L is mounted on said film J. Insulin ports B are drilled through casing A and cylinder L. Between film J and casing A a sponge M is provided and has the same function as a spring. When the glucose content in tissue fluid increases fluid will pass out through membrane D and an underpressure will result in F. This will cause film J and, thus, piston K to be extended opening for insulin delivery through ports B. When the glucose content decreases the opposite will occur. In this embodiment no check valve is needed the limited pressure in said reservoir due to the tension in the reservoir material securing sufficient drive pressure for delivery of insulin.

Figure 10 shows an embodiment where a resilient membrane D with a hollow piston N is provided at the "end" of casing A. In F said piston is enclosed by a seal O. When fluid migrates from F an underpressure will result in F and membrane D with piston N is forced inwards and through valve flaps P. Thus, insulin can flow through the void in said piston and out to the tissue fluid. The opposite will occur when the glucose content is reduced. In this embodiment no check valve is needed.

Figure 11 shows the same embodiment as Figure 10,but having a larger membrane surface.

Figure 12 shows an embodiment comprising a non-compressible membrane Q that is provided in connection with capillary tube R. From said capillary tube a flexible hose S extends to the end of membrane Q. Inside hose S a piston T with a bore U and V is provided. In case of fluid migration from F due to an increase of the glucose content in the tissue fluid hose S will bulge out and permit insulin delivery along said hose and through bores V and U. In case of a reduction of the glucose content the opposite will occur. No check valve is necessary in this case.

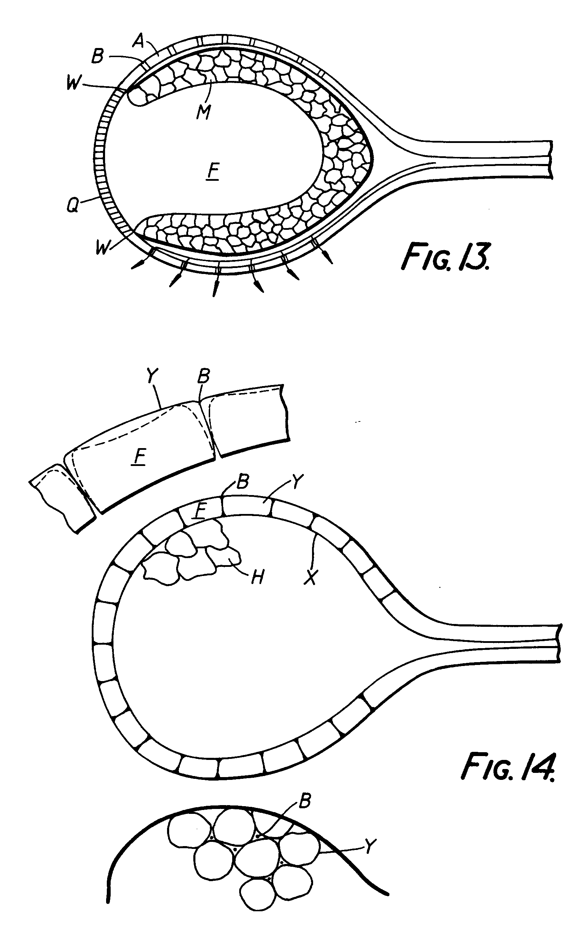

Figure 13 shows an embodiment with a casing A provided with a number of small ports B for insulin delivery. At the end of casing A a non-compressible membrane Q is provided. From the connection between casing A and membrane Q a resilient balloon is provided at W and is kept sealingly against the inside of casing A by the tension from a sponge M. In case of fluid transport from F due to increasing glucose content in the tissue fluid said balloon will collapse somewhat. Insulin can, then, flow along said balloon and the inside of casing A, and then through ports B. The opposite occurs when the glucose content in the tissue fluid decreases. No check valve is needed in this case either.

Figure 14 shows an embodiment comprising a non-resilient film X upon which cells Y are provided which cells are made semipermeable on top. Between cells ports B (openings) are provided for delivery of insulin. When the glucose content in the body is normal these ports are kept closed due to tensions in the cells, but when fluid migrates from F due to an increase of the glucose content in the tissue fluid said cells will "collapse" somewhat and open said ports. Insulin is, then, let out of sponge H. When the glucose content is reduced the opposite will occur. There is no need for a check valve here.

It applies to all embodiments that the indicator means, i.e. the membrane, is positioned in a place low in pressure in the human body. If this is in the abdominal cavity, it will be achieved at the same time that the insulin essentially will migrate through the liver in the same manner as insulin delivered by the pancreas in healthy people. This will enhance the controlling effect of the gland, because in this case it is to be expected that the so called late complications will be further reduced. Its positioning and the fact that tissue fluid will be formed around the membrane will prevent it in the embodiments according to Figures 3 and 4 from being subjected to exterior pressure causing uncontrolled amounts of insulin to flow out into the body. But even if this could happen in quite extraordinary cases, e.g. in case of a hard blow, only a small amount of insulin will be released and may be compensated, e.g. by intake of sugar. As regards the embodiments according to Figures 1, 6 and 7 even pressure cannot influence the membrane as it is not compressible. The same goes for the embodiment according to Figure 5, wherein the membrane is tight until it is activated by the glucose content of the body.

Nor should there be any hazard of pressure being exercised on the reservoir due to movement of muscles since fluid flow will be prevented by the safety means k and the capillary hole in hose g, or otherwise by the flexibility between said reservoir and muscle tissue.

Injection of the medicament according to the invention will occur in a manner analogous to the body functions, i.e. a signal is emitted to the gland in question to supply the body with medicament. In the disclosed case the gland is a pancreas and the medicament is insulin.

Besides using the gland for insulin injections as mentioned, it is also possible to use it for injections of all kinds of liquid medication that is dependent on certain changes in the blood composition, e.g. the content of cholesterol.

Another advantage of the invention is that it is not necessary to penetrate a vein in order to detect the glucose content. A foreign body in the blood-stream would be far more subjected to be clogged by proteine, and the like.

## Claims

1. An artificial gland for implantation in the human body comprising a medicament reservoir intended to be filled with a medicament solution by the aid of a syringe, wherein said reservoir is connected via a passage (g) to a casing, the wall of which is completely or partly comprised of a semi-permeable membrane (e, D) causing changes of pressure by osmosis for delivery of a medicament, **characterized in** that and said passage (g) includes a pressure activated valve (h) preventing uncontrolled amount of medicament to flow into the body, that a sponge (f, H) for absorbing medicament solution is enclosed in said casing, said body occupying only part of the casing void and being defined against the remaining portion of said void by a tight film, from which channels with outwardly opening one-way valves lead out through the semi-permeable membrane casing wall, the space between said casing wall and said tight film, in use, being filled with body fluid.

2. An artificial gland for implantation in the human body comprising a medicament reservoir intended to be filled with a medicament solution by the aid of a syringe, wherein said reservoir is connected via a passage (g) to a casing, the wall of which is completely or partly comprised of a semi-permeable membrane (e, D) causing changes of pressure by osmosis for delivery of a medicament, **characterized in** that the casing wall includes a rigid semi-permeable membrane (Q) and that a flexible hose (S) leads from the capillary tube end inside said casing to the opposite casing wall, said flexible hose enclosing a plug (T) having a blind centre bore (U), one end of which extends out through said casing wall, while its other end opens into a transverse bore (Y) covered by said flexible hose.

3. An artificial gland as defined in either of claims 1 or 2, **characterized in** that said passage further includes a one-way valve (k) opening towards the casing.

4. An artificial gland as defined in claim 1,
**characterized in** that said casing wall (e) consists of a rigid, semi-permeable membrane permitting a build-up of osmotic pressure in the body fluid in the space between said casing wall and said tight film to force medicament solution out of said sponge (f).

5. An artificial gland as defined in claim 1,
**characterized in** that part of said casing wall (A) consists of a resilient, semi-permeable membrane (D) provided with a portion (E) pressing against the film (G) enclosing said sponge (H) and liberating medicament solution therefrom, when said membrane collapses due to reduction of the osmotic pressure in the body fluid in the space between said casing wall and said tight film.

6. An artificial gland as defined in claim 4,
**characterized in** that the casing (A) is rigid and that its wall is perforated with small openings (B), which at one end are closed by a rigid, semi-permeable membrane (Q), a resilient balloon (w) being attached at the zone of transition between said perforated casing wall (A) and said membrane (Q), which balloon is biased into sealing contact with the inside of said perforated casing wall by the aid of the spring actions of a sponge (M) provided inside said balloon.

## Patentansprüche

1. Künstliche Drüse zur Implantation in den menschlichen Körper, bestehend aus einem Medikamentenreservoir, das mittels einer Spritze mit einer Medikamentenlösung füllbar ist; wobei das Reservoir über eine Kanal (g) mit einem Gehäuse verbunden ist, dessen Wand vollständig oder teilweise aus einer semipermeablen Membran (e,D) besteht, durch die das Medikament aufgrund osmose-bedingter Druckänderungen freisetzbar ist,
dadurch gekennzeichnet,
daß dar Kanal (g) ein durch Druck betätigbares Ventil (h) aufweist, durch das ein Einströmen einer unkontrollierten Menge des Medikaments in den Körper verhinderbar ist,
daß ein Schwamm (f,H) zur Absorption der Medikamentenlösung in dem Gehäuse enthalten ist, wobei dessen Körper nur einen Teil des Gehäusehohlraumes einnimmt und gegen den restlichen Teil des Hohlraumes durch einen festen Überzug abgegrenzt ist, aus dem Kanäle mit nach außen öffnenden Einwegventilen durch eine aus einer semipermeablen Membran bestehenden Gehäusewand führen, wobei der Raum zwischen der Gehäusewand und dem festen Überzug bei Gebrauch mit Körperflüssigkeit gefüllt ist.

2. Künstliche Drüse zur Implantation in den menschlichen Körper, bestehend aus einem Medikamentenreservoir, das mittels einer Spritze mit einer Medikamentenlösung füllbar ist; wobei das Reservoir über eine Kanal (g) mit einem Gehäuse verbunden ist, dessen Wand vollständig oder teilweise aus einer semipermeablen Membran (e,D) besteht, durch die das Medikament aufgrund osmose-bedingter Druckänderungen freisetzbar ist,
dadurch gekennzeichnet, daß die Gehäusewand eine steife semipermeable Membran (Q) aufweist, und
daß ein flexibler Schlauch (S) vom Ende der Kapillarröhre in dem Gehäuse zur gegenüberliegende Gehäusewand führt, wobei der flexible Schlauch einen Stopfen (T) mit einer zentrierten Blindbohrung (U) umschließt, deren eines Ende sich nach außen durch die Gehäusewand erstreckt, während sich dessen anderes Ende in eine vertikale Bohrung (V) mündet, die von dem flexiblen Schlauch abgedeckt ist.

3. Künstliche Drüse nach einem der Ansprüche 1 oder 2,
dadurch gekennzeichnet,
daß der Kanal weiterhin ein Einwegventil (k) aufweist, des sich in Richtung des Gehäuses öffnet.

4. Künstliche Drüse nach Anspruch 1,
dadurch gekennzeichnet,
daß die Gehäusewand (e) aus einer steifen, semipermeablen Membran besteht, die den Aufbau des osmotischen Drucks in der Körperflüssigkeit in dem Raum zwischen der Gehäusewand und dem festen Überzug derart erlaubt, daß die Medikamentenlösung aus dem Schwamm (f) preßbar ist.

5. Künstliche Drüse nach Anspruch 1,
dadurch gekennzeichnet,
daß ein Teil der Gehäusewand (A) aus einer elastischen, semipermeablen Membran (D), mit einem Abschnitt (E) besteht, der gegen den Überzug (G) drückt, der den Schwamm (H) umgibt, und die Medikamentenlösung daraus freisetzt, wenn sich die Membran aufgrund einer Verringerung des osmotischen Drucks in der Körperflüssigkeit im Raum zwischen der Gehäusewand und dem festen Überzug zusammenzieht.

6. Künstliche Drüse nach Anspruch 4,
dadurch gekennzeichnet,
daß das Gehäuse (A) steif, und seine Wand mit kleinen Öffnungen (B) perforiert ist, wobei das Gehäuse an einem Ende durch eine steife, semipermeable Membran (Q) geschlossen ist,
wobei ein elastischer Ballon (W) in der Übergangszone zwischen der perforierten Gehäusewand (A) und der Membran (Q) angebracht ist, der unter Zuhilfenahme der federnden Wirkung des sich innerhalb des Ballons befindlichen Schwamms (M) in abdichtendem Kontakt mit der Innenseite der perforierten umgebenden Gehäusewand vorgespannt ist.

## Revendications

1. Glande artificielle destinée à être implantée dans le corps humain, comprenant un réservoir à médicament destiné à être rempli d'une solution de médicament à l'aide d'une seringue, dans laquelle ledit réservoir est relié par un passage (g) à une enveloppe dont la paroi est constituée totalement ou partiellement par une membrane semiperméable (e, D) qui provoque des variations de pression par osmose pour la fourniture d'un médicament, caractérisée en ce que ledit passage (g) comprend une soupape (h) activée par la pression qui empêche une quantité incontrôlée de médicament de s'écouler dans le corps, en ce qu'une éponge (f, H) destinée à absorber la solution de médicament est enfermée dans ladite enveloppe, ledit corps occupant seulement une partie du vide de l'enveloppe et étant délimité par rapport à la partie restante dudit vide par un film étanche d'où des canaux comportant des soupapes à une voie débouchant vers l'extérieur conduisent à l'extérieur à travers la paroi de l'enveloppe constituée par la membrane semiperméable, l'espace compris entre ladite paroi de l'enveloppe et ledit film étanche étant, en fonctionnement, rempli de fluide corporel.

2. Glande artificielle destinée à être implantée dans le corps humain, comprenant un réservoir à médicament destiné à être rempli d'une solution de médicament à l'aide d'une seringue, dans laquelle ledit réservoir est relié par un passage (g) à une enveloppe dont la paroi est totalement ou partiellement constituée par une membrane semiperméable (e, D) provoquant des variations de pression par osmose pour la fourniture d'un médicament, caractérisée en ce que la paroi de l'enveloppe comprend une membrane semiperméable rigide (Q) et en ce qu'un tuyau flexible (S) conduit de l'extrémité d'un tube capillaire à l'intérieur de ladite enveloppe à la paroi opposée de l'enveloppe, ledit tuyau flexible entourant un bouchon (T) comportant un orifice central aveugle (U) dont une extrémité s'étend vers l'extérieur à travers ladite paroi de l'enveloppe, tandis que son autre extrémité débouche dans un orifice transversal (Y) recouvert par ledit tuyau flexible.

3. Glande artificielle selon l'une quelconque des revendications 1 et 2, caractérisée en ce que ledit passage comprend en outre une soupape à une voie (K) qui débouche vers l'enveloppe.

4. Glande artificielle selon la revendication 1, caractérisée en ce que ladite paroi (E) de l'enveloppe consiste en une membrane semiperméable rigide qui permet l'établissement d'une pression osmotique dans le fluide corporel dans l'espace compris entre ladite paroi de l'enveloppe et ledit film étanche pour forcer la solution de médicament hors de ladite éponge (F).

5. Glande artificielle selon la revendication 1, caractérisée en ce qu'une partie de ladite paroi (A) de l'enveloppe consiste en une membrane semiperméable élastique (D) munic d'une partie (E) qui exerce une pression contre le film (G) qui entoure ladite éponge (H) et qui en libère la solution de médicament lorsque ladite membrane s'affaisse par suite d'une réduction de la pression osmotique dans le fluide corporel dans l'espace compris entre ladite paroi de l'enveloppe et ledit film étanche.

6. Glande artificielle selon la revendication 4, caractérisée en ce que l'enveloppe (A) est rigide et en ce que sa paroi est perforée de petites ouvertures (B) qui, à une extrémité, sont fermées par une membrane semiperméable rigide (Q), un ballon élastique (W) étant fixé à la zone de transition entre ladite paroi perforée (A) de l'enveloppe et ladite membrane (Q), lequel ballon est poussé en contact étanche avec l'intérieur de ladite paroi perforée de l'enveloppe à l'aide des actions de ressort d'une éponge (M) prévue à l'intérieur dudit ballon.
